(19) Europäisches Patentamt / European Patent Office / Office européen des brevets

(11) **EP 4 485 258 B1**

(12) **EUROPEAN PATENT SPECIFICATION**

(45) Date of publication and mention
of the grant of the patent:
**24.12.2025   Bulletin 2025/52**

(51) International Patent Classification (IPC):
**G16B 50/50** (2019.01)     **G06F 21/62** (2013.01)
**G16B 50/40** (2019.01)     **H04L 9/00** (2022.01)

(21) Application number: **24182251.9**

(52) Cooperative Patent Classification (CPC):
**G06F 21/6245; G16B 50/40; G16B 50/50; H04L 9/008**

(22) Date of filing: **14.06.2024**

(54) **METHOD AND SYSTEM TO COMPUTE AN ABUNDANCE HISTOGRAM IN A PRIVACY PRESERVING MANNER**

VERFAHREN UND SYSTEM ZUR BERECHNUNG EINES HÄUFIGKEITSHISTOGRAMMS AUF EINE DATENSCHUTZERHALTENDE WEISE

PROCÉDÉ ET SYSTÈME DE CALCUL D'HISTOGRAMME D'ABONDANCE DE MANIÈRE PRÉSERVANT LA CONFIDENTIALITÉ

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:   **27.06.2023   IN 202321043119**

(43) Date of publication of application:
**01.01.2025   Bulletin 2025/01**

(73) Proprietor: **Tata Consultancy Services Limited
Maharashtra (IN)**

(72) Inventors:
• **NARUMANCHI, Harika**
  **500081 Hyderabad, Telangana (IN)**
• **EMMADI, Nitesh**
  **500081 Hyderabad, Telangana (IN)**
• **SAGLANI, Divyesh**
  **411057 Pune - Maharashtra (IN)**
• **ALASINGARA BHATTACHAR, Rajan Mindigal**
  **560009 Bangalore - Karnataka (IN)**
• **SIVADASAN, Naveen**
  **500081 Hyderabad, Telangana (IN)**

(74) Representative: **Goddar, Heinz J.
Boehmert & Boehmert
Anwaltspartnerschaft mbB
Pettenkoferstrasse 22
80336 München (DE)**

(56) References cited:
• **NAVEEN SIVADASAN ET AL: "Kmerlight: fast and accurate k-mer abundance estimation", ARXIV.ORG, CORNELL UNIVERSITY LIBRARY, 201 OLIN LIBRARY CORNELL UNIVERSITY ITHACA, NY 14853, 19 September 2016 (2016-09-19), XP080727579**
• **ABINAYA B ET AL: "A survey on genomic data by privacy-preserving techniques perspective", COMPUTATIONAL BIOLOGY AND CHEMISTRY, ELSEVIER, AMSTERDAM, NL, vol. 93, 29 June 2021 (2021-06-29), XP086711636, ISSN: 1476-9271, [retrieved on 20210629], DOI: 10.1016/ J.COMPBIOLCHEM.2021.107538**

## Description

CROSS-REFERENCE TO RELATED APPLICATIONS AND PRIORITY

The present application claims priority from Indian patent application number 202321043119, filed on June 27, 2023.

TECHNICAL FIELD

The disclosure herein generally relates to the field of preserving privacy of a genome, and, more particularly, to a method and system for computing *k-mer* abundance histogram on fully homomorphic encrypted genomic data in a privacy preserving manner.

BACKGROUND

A genome is an organism's complete set of Deoxyribonucleic Acid (DNA), that includes enormous genetic information. The genome is a collection of DNA molecules storing genetic information in a cell. It can be represented as a set of long strings over the alphabet (each string corresponding to one chromosome). In the DNA sequencing experiment, many reads are produced from the genome. These reads are short substrings obtained from random locations of the genome. Genome size and some other characteristics estimates are computed from a summary statistic of reads i.e., a *k-mer* abundance histogram. A *k-mer* is a substring of length exactly *k* and the histogram summarizes the number of occurrences of individual *k-mers* in the input set of reads. The histogram gives insights on the approximate total number of distinct k-mers and total number of k-mers with some multiplicity (frequency) while preserving the privacy of critical genomic data.

*K-mer* abundance histogram estimation is a critical constituent and has several applications in genome analysis. For instance, k-mer abundance information in sequence data is useful in read error correction, parameter estimation for genome assembly, digital normalization, sampling error rate etc. However, existing *k-mer* abundance estimation techniques do not address the problem of data privacy. Data privacy is important especially in fields like genome analysis which involve sensitive data of a subject such as genetic testing information like DNA or any personal health information, which when leaked results in irreversible damage for the subject. Further, with the growing use of large-scale datasets containing the subject's genomics and clinical data for research and studies purposes, it is important to ensure the privacy of the subject by generating a secure representation of genome data observation. NAVEEN SIVADASAN ET AL: "Kmerlight: fast and accurate k-mer abundance estimation",ARXIV.ORG, CORNELL UNIVERSITY LIBRARY, 201 OLIN LIBRARY CORNELL UNIVERSITY ITHACA, NY 14853, 19 September 2016 teaches computing a k-mer abundance histogram from genomic sequence data using the kmerlight algorithm, but does not attempt to perform this calculation in a privacy-preserving manner.

SUMMARY

Embodiments of the present disclosure present technological improvements as solutions to one or more of the above-mentioned technical problems recognized by the inventors in conventional systems. The invention is set out in appended set of claims.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, explain the disclosed principles:

FIG. 1 illustrates an exemplary system for computing abundance histogram on fully homomorphic encrypted genomic data in a privacy preserving manner, according to some embodiments of the present disclosure.
FIG. 2 is a flow diagram to illustrate an overview of privacy preserving genomics, according to some embodiments of the present disclosure.
FIGS. 3A and 3B (collectively referred as FIG. 3) are exemplary flow diagrams illustrating a processor-implemented method for computing abundance histogram on a fully homomorphic encrypted genomic data in a privacy preserving manner, according to some embodiments of the present disclosure.
FIG. 4 is a flow diagram to illustrate an overview of kmerlight algorithm, according to some embodiments of the present disclosure.

FIG. 5A and 5B (collectively referred to as FIG. 5) are schematic diagrams to illustrate a comparison of original $F_i$ function and the approximated $F_i$ function, according to some embodiments of the present disclosure.

FIGS. 6A and 6B (collectively referred as FIG. 6) are schematic diagrams to illustrate an error analysis in logarithmic approximation, according to some embodiments of the present disclosure.

## DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

A genome is typically a string of nucleotides (Adenine (A), Cytosine (C), Guanine (G), and Thymine (T)). For example, ATGCGTCTC......G. Genomes are generally large biological sequence strings, and these are rendered into a stream of substrings of suitable length called *k-mers* and are used for computational genomics and sequence analysis. One such critical analysis is k-mer abundance estimation. Given an input genome sequence, *k-mer* abundance estimation algorithm provides estimations such as the total number of distinct *k-mers* in the sequence and total number of *k-mers* with frequency/multiplicity *i* (referred to as $F_i$). There are several approaches in literature that can compute these estimates. However, these approaches do not address the privacy of the genome sequences.

Embodiments herein provide a method and system for computing abundance histogram on a fully homomorphic encrypted genomic data in a privacy preserving manner. The system is configured to estimate abundance of nucleotide strings on the encrypted nucleotide strings using a privacy preserving kmerlight algorithm. Kmerlight is a streaming algorithm, which in general, processes a sequence of *k-mers* in a single pass using only a limited amount of memory and time. The kmerlight maintains an approximate summary, or a sketch, of the previously viewed *k-mers* and with each new *k-mer* the sketch is updated. When all the *k-mers* are processed, the sketch can be analyzed to provide the estimate of the *k-mer* abundance histogram. Kmerlight combines the techniques of sampling and hashing to maintain a sketch of *k-mers* and from the contents of the sketch computes an estimate of the histogram.

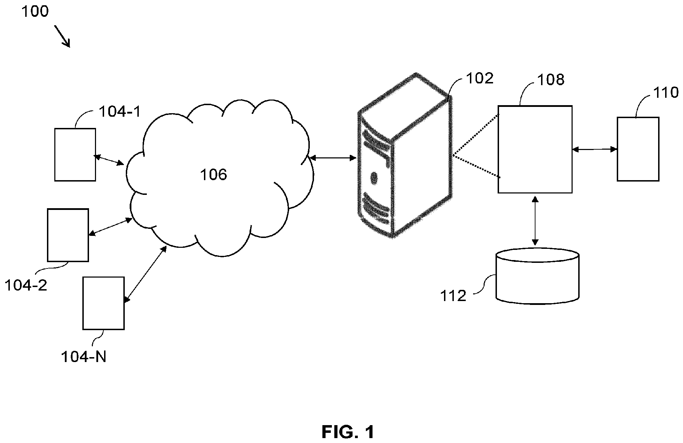

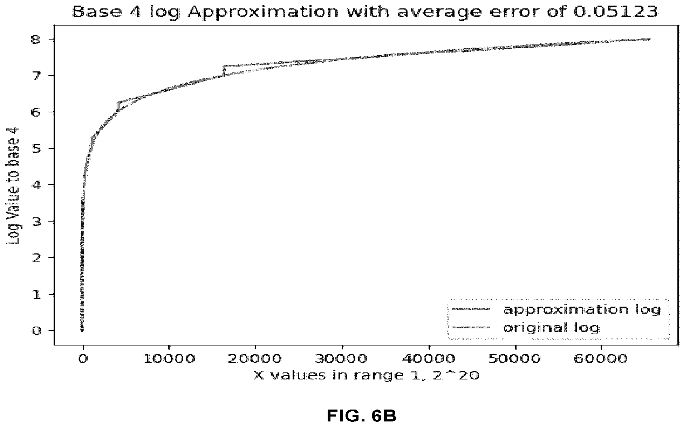

Referring now to the drawings, and more particularly to FIG. 1 through FIGS. 6A and 6B, where similar reference characters denote corresponding features consistently throughout the figures, there are shown preferred embodiments and these embodiments are described in the context of the following exemplary system and/or method.

FIG. 1 illustrates a block diagram of a system 100 for computing abundance histogram on a fully homomorphic encrypted genomic data in a privacy preserving manner, in accordance with an example embodiment. Although the present disclosure is explained considering that the system 100 is implemented on a server, it may be understood that the system 100 may comprise one or more computing devices 102, such as a laptop computer, a desktop computer, a notebook, a workstation, a cloud-based computing environment and the like. It will be understood that system 100 may be accessed through one or more input/output interfaces 104-1, 104-2... 104-N, collectively referred to as I/O interface 104. Examples of the I/O interface 104 may include, but are not limited to, a user interface, a portable computer, a personal digital assistant, a handheld device, a smartphone, a tablet computer, a workstation, and the like. The I/O interface 104 is communicatively coupled to the system 100 through a network 106.

In an embodiment, the network 106 may be a wireless or a wired network, or a combination thereof. In an example, the network 106 can be implemented as a computer network, as one of the different types of networks, such as virtual private network (VPN), intranet, local area network (LAN), wide area network (WAN), the internet, and such. The network 106 may either be a dedicated network or a shared network, which represents an association of the different types of networks that use a variety of protocols, for example, Hypertext Transfer Protocol (HTTP), Transmission Control Protocol/Internet Protocol (TCP/IP), and Wireless Application Protocol (WAP), to communicate with each other. Further, network 106 may include a variety of network devices, including routers, bridges, servers, computing devices, storage devices. The network devices within network 106 may interact with the system 100 through communication links.

The system 100 supports various connectivity options such as BLUETOOTH®, USB, ZigBee, and other cellular services. The network environment enables connection of various components of the system 100 using any communication link including Internet, WAN, MAN, and so on. In an exemplary embodiment, the system 100 is implemented to operate as a stand-alone device. In another embodiment, the system 100 may be implemented to work as a loosely coupled device to a smart computing environment. Further, the system 100 comprises at least one memory with a plurality of instructions, one or more databases 112, and one or more hardware processors 108 which are communicatively coupled with the at least one memory to execute a plurality of modules 114 therein. The components and functionalities of the system 100 are described in further detail.

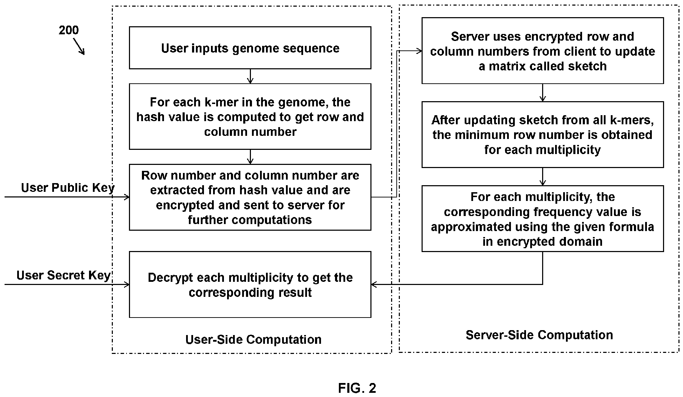

FIG. 2 is a schematic diagram 200 illustrating a privacy preserving genomics implemented by the system 100 of FIG. 1. A user wishes to use a DNA analysis service provided by a service provider, however the user does not want to disclose its genetic information to the service provider. Embodiments herein provide a system and method to perform DNA analysis on an encrypted genome without the need for decryption and ensures user's genome privacy. Herein, a stream of input genome is divided into chunks called as the sketches where each k-mer in the sketch is hashed to get a sampling level and a counter value for the k-mer in a sketch. The sampling level and counter value are used to update the *k-mer* position in a global matrix that is created for the single input genome. Using the global matrix, number of distinct *k-mers and k-mers* with multiplicity *i* are computed.

Further, a privacy enhancing technique is designed over the representations using a Fully Homomorphic Encryption (FHE). Genomic data is analyzed with identified efficient algorithms for real world deployment in encrypted domain. Analysis of *k-mers,* which are nucleotide strings of length *k* present in a genome sequence, is one of the fundamental operations in computational genomics. In particular, the problem of computing *k-mer* abundance estimation is considered in a genomic sequence.

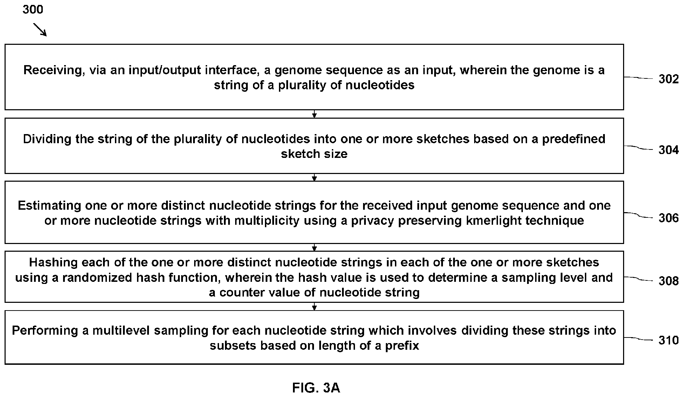

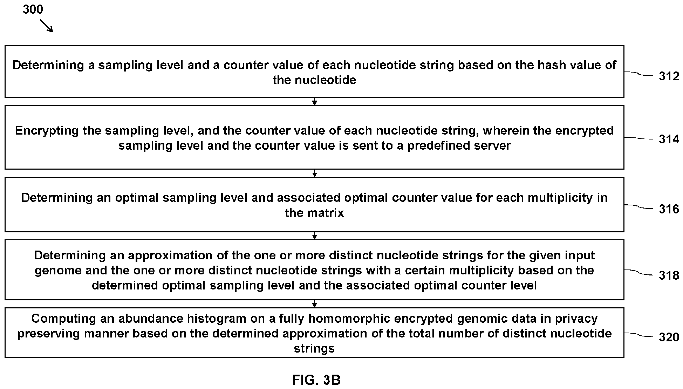

FIGS. 3A and 3B (collectively referred as FIG. 3) are flow diagrams illustrating a processor-implemented method 300 for computing abundance histogram on a fully homomorphic encrypted genomic data in a privacy preserving manner implemented by the system 100 of FIG. 1. Functions of the components of the system 100 are now explained with reference to FIG. 2 through steps of flow diagram in FIG. 3, according to some embodiments of the present disclosure.

Initially, at step 302 of the processor-implemented method 300, one or more hardware processors 108 are configured by the programmed instructions to receive a genome sequence as an input. The genome is a string of a plurality of nucleotides also called as *k-mers.* Hereinafter, the string of a plurality of nucleotides is used interchangeably as *k-mers.*

At the next step 304 of the processor-implemented method 300, the one or more hardware processors 108 are configured by the programmed instructions to divide the string of the plurality of nucleotides into one or more sketches based on a predefined sketch size. In the sketching technique the input genome is subdivided into a series of chunks defined as sketches. Sketching is a method used in getting a simplified and summarized representation of large data sets that preserves important information while discarding the unnecessary details in a stream of data.

In one example, a user has a large stream of numbers, and the user wants to compute average value of the entire list. The system is configured to generate reads from the bigger stream of numbers and create a sketch for each read that stores only few representation numbers from the bigger list. The system can compute average of each sketch and use these averages to compute the overall average that can provide a rough average estimate for the entire list.

Each sketch has a state matrix of a level and a counter with the size which is configurable such as level 64 and the counter=$2^{20}$. Each cell of the state matrix is updated. Further, the level number and counter value for each *k-mer* is obtained from the hash function in a sketch. The cell corresponding to this level number and counter number is then updated for the state matrix after checking if there exists no collision, where collision is a property where two *k-mers* have same level and counter number.

At the next step 306 of the processor-implemented method 300, the one or more hardware processors 108 are configured by the programmed instructions to estimate one or more distinct *k-mers* for the received input genome sequence and one or more *k-mers* with multiplicity using a privacy preserving kmerlight technique. The one or more distinct *k-mers* i.e., $F_0$ is computed as follows:

$$F_0 = 2^{w-optimal} * \frac{\log\left(\frac{t-optimal}{R}\right)}{\log\left(1-\frac{1}{R}\right)} \tag{1}$$

wherein, w is the level number, which ranges from 0... 64, *w-optional* is a level that has the number of counters with maximum count with a multiplicity *i, t* is the counter value which ranges from 0...$2^{20}$, and *t-optimal* is computed as max (*number of levels - number of columns with multiplicity i*). All the $F_0$ and $F_i$ are performed in encrypted domain, wherein *t*-optimal, *w*-optimal, $F_0$ and $F_i$ are encrypted and *r* is unencrypted and constant. The one or more *k-mers* with multiplicity *i* is computed as follows:

$$F_i = \frac{2^{w-optimal} * \left(\left(\frac{t-1}{R}\right) * t-optimal\right)}{(1-\frac{1}{R})^{\frac{F_0}{2^{w-optimal}}}} \tag{2}$$

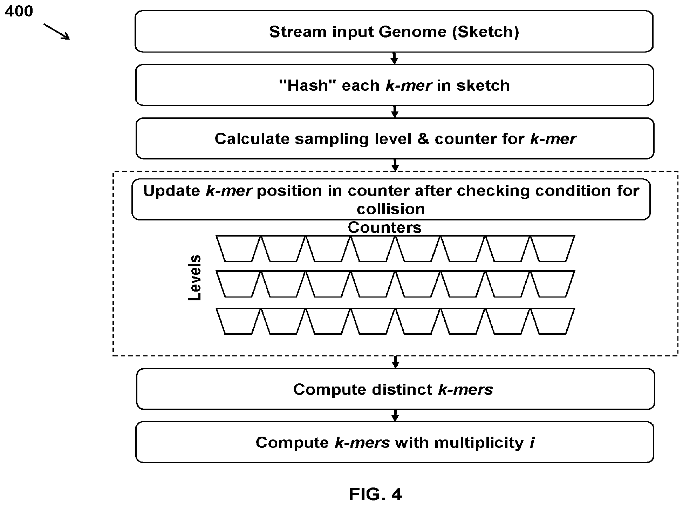

The one or more distinct *k-mers* are estimated using approximation formula provided, and to approximate nucleotides with multiplicity *i*, an optimal sampling level and counter number is determined from the sketch matrix and these numbers are used in the approximation formula to get the result as shown in FIG. 4. The optimal sampling level for a multiplicity *i* is the lowest level where maximum number of *k-mers* with multiplicity *i* are present. The optimal counter value is the maximum number of counters with multiplicity *i* from all levels.

At the next step 308 of the processor-implemented method 300, the one or more hardware processors 108 are configured by the programmed instructions to hash each of the one or more distinct *k-mers* in each of the one or more sketches using a randomized hash function. Wherein the hash value is used to determine a sampling level and a counter value of *k-mer*. Each input *k-mer* is hashed using a randomized hash function: *hash_val ← H(k - mer)*.

Each *k-mer* can be hashed using any secure randomized hash function. In one aspect, the hash function used by kmerlight algorithm is *murmur3* hash. It is non-cryptographic hash function suitable for general hash-based lookup table. *Murmur3* hash yields a 32-bit or 128-bit hash values. It is optimized based on the system architecture.

At the next step 310 of the processor-implemented method 300, the one or more hardware processors 108 are configured by the programmed instructions to perform a multilevel sampling for each nucleotide string which involves dividing these strings into subsets based on length of a prefix, wherein the prefix comprises one or more trailing zeros and one or more leading zeros in the hash value of nucleotide. In multi-level sampling, *k-mers* are segregated into levels, based on the length of the prefix say *w*, of hash values. For each level *w*, counters are maintained that determine the number of times each k-mer has occurred. For example, sampling level (*w*) = 1+ number of trailing zeros in binary representation of hash;

$$\text{counter value} = \text{floor} \left( \frac{x}{u} \right) \bmod r$$ , where *r* and *u* are predefined constants with values by the user. Wherein, *r* is sketch size and *u* is any constant value.

$$x = \text{hash value}/2^w$$

where w is the sampling level.

At the next step 312 of the processor-implemented method 300, the one or more hardware processors 108 are configured by the programmed instructions to determine a sampling level and a counter value of each nucleotide string based on the hash value of the nucleotide, wherein a matrix of the sampling level and counter value is prepared for each of the one or more sketches. In the hash value for a *k-mer* obtained from murmur3 hash function, the number of trailing zeros in the hash value will give the sampling level number. The counter number is computed as:

$$x = \text{hash value} / 2^w,$$

where w is the sampling level obtained from above step and

$$\text{counter number} = \text{floor}(x/u) \bmod r,$$

where *r* and *u* are predefined constants with values by the user.

At the next step 314 of the processor-implemented method 300, the one or more hardware processors 108 are configured by the programmed instructions to encrypt the sampling level and the counter value of each nucleotide string. The encrypted sampling level and the counter value is sent to a predefined server, wherein the predefined server checks condition for collision for the encrypted input data to update the matrix of the sampling level and counter value.

Note that the state-of-the-art data structures can handle only encryption of larger integers by increasing the parameter size. In data heavy applications, increasing the parameter size will make the application inefficient. In the present disclosure, a data structure called *BigCipher* is suitable for encrypting large integers, with a minimal parameter set. The *BigCipher* is like a big integer structure, a radix-based implementation in base *b*, which can accommodate large integer encryption. The *BigCipher* is designed to overcome the limitations existing in the current FHE library, that can only accommodate encryption of short integers. This data structure is especially important in the applications dealing with streaming inputs of larger sizes that need to be encrypted.

Further, the one or more hardware processors 108 are configured by the programmed instructions to approximate logarithm and exact computation of exponentiation of 2 in encrypted domain which can compute exponentiation of large integers without increasing the parameter set. This exponentiation function uses the idea of shifting. Usually, in FHE libraries, when exponentiation is computed for larger integers, the parameter sets need to be increased, which in turn impacts the performance adversely. This hinders performance and becomes inefficient as the size of integer grows. However, in the present disclosure, function can compute exponentiation of large integers without increasing the parameter sets. The input is encoded using base-4 encoding and independently encrypted into a *BigCipher* data structure. Herein, an encrypted index of the first non-zero coefficient of the input in the *BigCipher* is extracted. For example, there is a number n=123, representation of n in base '4' is shown below (size of 16 bits, which is enough to support $4^{16}-1$ numbers):

5

$$123 = 64 + 48 + 8 + 3 = 0000000000001323,$$

each bit of the above
representation is stored in the form of a vector, 0000010100101011 which as a whole represents a *BigCipher.*

For FHE, polynomial approximation is used and then approximation is applied. To compute non-linear function over FHE, the state of the art techniques uses polynomial approximations like taylor-series approximation technique. The main drawback of this technique is that it is required to know the range of input, because the input has to be scaled to a smaller range for more accurate approximations. One such use case if of logarithm approximation, where input is needed to be scaled down to (0,1). Using *bigCipher*, the system doesn't need to scale down the input however large it may be and yet the system can still compute logarithm approximation with an average error of 0.05 and maximum error of 0.25. It is also important to note that this logarithm approximation algorithm provided by us is applied with small FHE parameter set and provides a much better accuracy than polynomial approximation methods for large integers. It would be appreciated that it can work with a small FHE parameter set and is accurate for large integers. Logarithmic approximation with an average error of 0.05 and a maximum error of 0.25.

At the next step 316 of the processor-implemented method 300, one or more hardware processors 108 are configured by the programmed instructions to determine an optimal sampling level and associated optimal counter value for each multiplicity in the matrix. The optimal sampling level is the level which has the most occurrence of a multiplicity in the matrix.

To compute the optimal sampling level following steps are performed. *w*-optimal is the optimal sampling level and *t*-optimal is the optimal counter value with initial value as 0. *t* is used to compute *t*-optimal, wherein *t* = number of *k-mers* with multiplicity *i* present in a row. For each sampling level *w*, if *t* > *t*-optimal, *w*-optimal = *w*, *t*-optimal = *t*. Using these last *t*-optimal and *w*-optimal values, the system is configured to compute the $F_0$ and $F_i$ values.

At the next step 318 of the processor-implemented method 300, the one or more hardware processors 108 are configured by the programmed instructions to determine an approximation of the one or more distinct *k-mers* for the given input genome and the one or more *k-mers* with a certain multiplicity based on the determined optimal sampling level and an associated optimal counter level as shown in Table 1.

**Table 1**

| Operation | Time for *BigCipher* | Time for Plain Computation (using direct operations) |
|---|---|---|
| Addition | 583.19 ms | 230.0 ns |
| Logarithm | 15.41 s | 60.0 ns |
| Exponentiation | 16.67 s | 70.0 ns |
| Mux | 1.27 s | 60.0 ns |
| Binary Multiplication | 112.97 ms | 60.0 ns |
| Number Comparison | 3.36 s | 70.0 ns |

Finally, at the last step 320 of the method 300, the one or more hardware processors 108 are configured by the programmed instructions to compute an abundance histogram on a fully homomorphic encrypted genomic data in privacy preserving manner based on the determined approximation of the total number of distinct nucleotide strings. Fully Homomorphic Encryption (FHE) enables computations on encrypted data without the need for decryption, thereby preserving privacy of the input data. For a set of FHE ciphertext corresponding to a set of plaintexts, any arbitrary function can be evaluated without revealing the plaintexts. FHE supports addition and multiplication as primitive operations:

$$Enc(a + b) = Enc(a) + Enc(b) \tag{3}$$

$$Enc(a + b) = Enc(a) + Enc(b) \tag{4}$$

A public key FHE scheme $\xi$ consists of an additional eval$_\xi$ along with the usual (KeyGen$_\xi$, Enc$_\xi$, Dec$_\xi$) from any other public key scheme. Eval$_\xi$ is the evaluation algorithm used for computations on encrypted data. This algorithm takes as input a polynomial expression *P* and a set of ciphertext sc = {$C_0$, $C_1$, ..., $C_n$} as inputs to *P*. The input output of eval$_\xi$ satisfies following equation:

$$Dec_\xi\big(\mathrm{Eval}_\xi(P,c,pk),sk\big) = P(Dec_\xi(c,sk)) \qquad (5)$$

To improve the efficiency of the homomorphic operations and to reduce space complexity, one can leverage homomorphic batching technique where multiple plaintext is batched into a single ciphertext. On this batched ciphertext, the operations can be performed on component wise plaintexts and can be executed parallelly in single Instruction Multiple Data (SIMD) manner.

For a radix-based implementation of a number in base $b$, the logarithm computes the log value of the number in base '$b$'. Logarithm is expressed as sum of mantissa and exponent, where mantissa is the integer part and exponent are the decimal part. To compute mantissa, the index of the last non-zero coefficient of the radix representation of number is find in zero based indexing. To compute the decimal, the values from the last non-zero index is copied to evaluate it as in base $b$ representation of decimal number. To compute log($n$) in base $4$:

$$\log(x) = \mathrm{mantissa} + \mathrm{exponent}$$

mantissa = index of first non-zero index from left (or index of last non-zero element from right) = 3 exponent = number from the first non-zero element represented as decimal representation for base b = 1323000000000000 the base 10 representation for above result is = 3 + (1*(1/4) + 3 *(1/16) + 2 *(1/64) + .....) = 3.48

$$\text{actual log value for } \log(123)/\log(4) = 3.471$$

**Experiment**

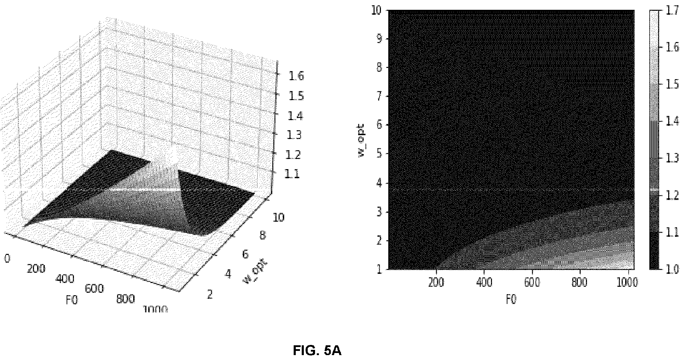

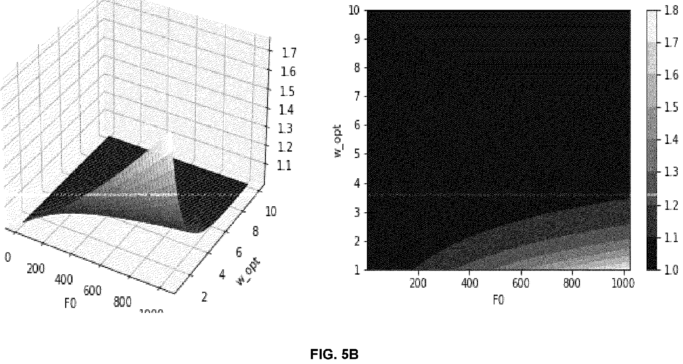

FIG. 5A and 5B are schematic diagrams to illustrate a comparison of original $F_i$ function and the approximated $F_i$ function, according to some embodiments of the present disclosure. The graph compares the original $F_i$ equation in kmerlight algorithm with the approximation algorithm in the privacy preserving kmerlight. The approximation equation has a minimal loss and accuracy is above 96%.

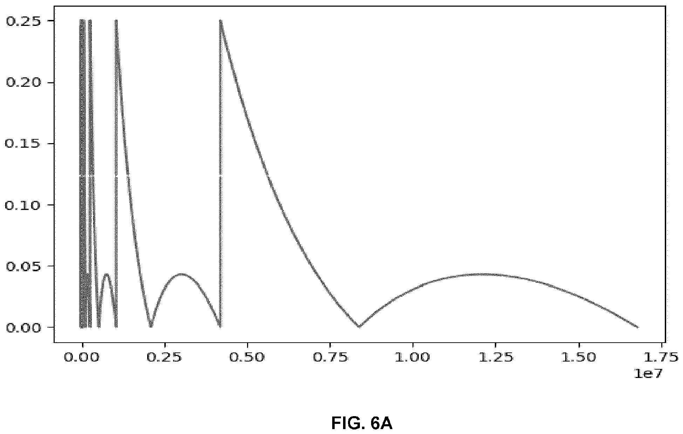

FIGS. 6A and 6B (collectively referred as FIG. 6) are schematic diagrams to illustrate an error analysis in logarithmic approximation, according to some embodiments of the present disclosure. In the FIG. 6A, the graph provides range of possible error values for the logarithmic approximation function. The X-axis represents the inputs values in the range 1 to $2^{20}$ and $Y$-axis represents the error between original logarithm and approximated logarithm. FIG. 6B provides the comparison of the original logarithm with the approximation of logarithm. The $X$-axis represents the inputs values in the range 1 to $2^{20}$ and $Y$-axis represents the output of logarithmic computation with an average error of 0.05.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of the present disclosure herein address unresolved problems of data privacy especially in genome analysis which involve sensitive data of a subject such as genetic testing information. This disclosure relates generally to a method and system for computing abundance histogram on fully homomorphic encrypted genomic data in a privacy preserving manner. privacy enhancing technique is designed over the representations using a Fully Homomorphic Encryption. Genomic data is analyzed with identified efficient algorithms for real world deployment in encrypted domain. Analysis of k-mers, which are nucleotide strings of length k present in a genome sequence is one of the fundamental operations in computational genomics. In particular, the problem of computing k-mer abundance estimation is considered in a genomic sequence.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor-implemented method (300), comprising:

    receiving (302), via an input/output interface (104), a genome sequence as an input, wherein the genome is a string of a plurality of nucleotides, by a client;
    dividing, via one or more hardware processors, the string of the plurality of nucleotides into one or more sketches based on a predefined sketch size, by the client;
    estimating (304), via the one or more hardware processors, one or more distinct nucleotide strings for the received input genome sequence and one or more nucleotide strings with multiplicity using a privacy preserving kmerlight technique, by the client;
    hashing (306), via the one or more hardware processors, each of the one or more distinct nucleotide strings in each of the one or more sketches using a randomized hash function, by the client, wherein a hash value is used to determine a sampling level and a counter value of a nucleotide string;
    performing (308), via the one or more hardware processors, a multilevel sampling for each nucleotide string which involves dividing these strings into subsets based on the length of a prefix, by the client;
    determining (310), via the one or more hardware processors, a sampling level and a counter value of each nucleotide string based on the hash value of the nucleotide string, by the client;
    encrypting (312), via the one or more hardware processors, the sampling level, and the counter value of each nucleotide string, by the client, wherein the encrypted sampling level and the counter value are sent to a predefined server;
    preparing a matrix of the encrypted sampling level and the counter value for each of the one or more sketches by the predefined server, wherein the matrix is updated using the encrypted sampling level and counter value for each nucleotide string in each sketch among the one or more sketches;

determining (314), via the one or more hardware processors, an optimal sampling level and an associated optimal counter value for each multiplicity in the matrix, by the predefined server;

determining (316), via the one or more hardware processors, an approximate count of the one or more distinct nucleotide strings for the given input genome and the one or more distinct nucleotide strings with a certain multiplicity based on the determined optimal sampling level and the associated optimal counter level, by the predefined server; and

computing (318), via the one or more hardware processors, an abundance histogram on fully homomorphic encrypted genomic data in a privacy preserving manner based on the determined approximation of the total number of distinct nucleotide strings.

2. The processor-implemented method (300) as claimed in claim 1, wherein the prefix comprises one or more trailing zeros and one or more leading zeros in the hash value of the nucleotide string.

3. The processor-implemented method (300) as claimed in claim 1, wherein the predefined server checks for a collision condition for the encrypted input data to update the matrix of the sampling level and counter value.

4. The processor-implemented method (300) as claimed in claim 1, wherein the optimal sampling level is the level which has the highest occurrence of the multiplicity in the matrix.

5. A system (100) comprising:

a plurality of input/output interfaces (104) to receive a genome sequence as an input, wherein the genome is a string of a plurality of nucleotides by a client;

a memory (110) in communication with the one or more hardware processors (108), wherein the one or more hardware processors are configured to execute programmed instructions stored in the memory (110) to:

divide the string of the plurality of nucleotides into one or more sketches based on a predefined sketch size, by the client;

estimate one or more distinct nucleotide strings for the received input genome sequence and one or more nucleotide strings with multiplicity using a privacy preserving kmerlight technique, by the client;

hash each of the one or more distinct nucleotide strings in each of the one or more sketches using a randomized hash function, by the client, wherein a hash value is used to determine a sampling level and a counter value of a nucleotide string:

perform a multilevel sampling for each nucleotide string which involves dividing these strings into subsets based on the length of a prefix, by the client;

determine a sampling level and a counter value of each nucleotide string based on the hash value of the nucleotide string, by the client;

encrypt the sampling level and the counter value of each nucleotide string, by the client, wherein the encrypted sampling level and the counter value are sent to a predefined server;

prepare a matrix of the encrypted sampling level and the counter value for each of the one or more sketches by the predefined server, wherein the matrix is updated using the encrypted sampling level and counter value for each nucleotide string in each sketch among the one or more sketches;

determine an optimal sampling level and an associated optimal counter value for each multiplicity in the matrix, wherein the optimal sampling level is the level which has the highest occurrence of a multiplicity in the matrix, by the predefined server;

determine an approximation of the one or more distinct nucleotide strings for the given input genome and the one or

more distinct nucleotide strings with a certain multiplicity based on the determined optimal sampling level and the associated optimal counter level, by the predefined server; and

compute an abundance histogram on fully homomorphic encrypted genomic data in a privacy preserving manner based on the determined approximation of the total number of distinct nucleotide strings.

6. The system (100) as claimed in claim 5, wherein the prefix comprises one or more trailing zeros and one or more leading zeros in the hash value of the nucleotide string.

7. The system (100) as claimed in claim 5, wherein the predefined server checks for a collision condition for the encrypted input data to update the matrix of the sampling level and counter value.

8. One or more non-transitory machine-readable information storage mediums comprising one or more instructions which when executed by one or more hardware processors cause:

receiving, via an input/output interface (104), a genome sequence as an input, wherein the genome is a string of a plurality of nucleotides, by a client;

dividing, via one or more hardware processors, the string of the plurality of nucleotides into one or more sketches based on a predefined sketch size, by the client;

estimating one or more distinct nucleotide strings for the received input genome sequence and one or more nucleotide strings with multiplicity using a privacy preserving kmerlight technique, by the client;

hashing each of the one or more distinct nucleotide strings in each of the one or more sketches using a randomized hash function, by the client, wherein a hash value is used to determine a sampling level and a counter value of a nucleotide string;

performing a multilevel sampling for each nucleotide string which involves dividing these strings into subsets based on the length of a prefix, by the client;

determining a sampling level and a counter value of each nucleotide string based on the hash value of the nucleotide string, by the client;

encrypting the sampling level, and the counter value of each nucleotide string, by the client, wherein the encrypted sampling level and the counter value are sent to a predefined server;

preparing a matrix of the encrypted sampling level and the counter value for each of the one or more sketches by the predefined server, wherein the matrix is updated using the encrypted sampling level and counter value for each nucleotide string in each sketch among the one or more sketches;

determining an optimal sampling level and an associated optimal counter value for each multiplicity in the matrix, wherein the optimal sampling level is the level which has the highest occurrence of a multiplicity in the matrix, by the predefined server;

determining an approximate count of the one or more distinct nucleotide strings for the given input genome and the one or more distinct nucleotide strings with a certain multiplicity based on the determined optimal sampling level and the associated optimal counter level, by the predefined server; and

computing an abundance histogram on fully homomorphic encrypted genomic data in a privacy preserving manner based on the determined approximation of the total number of distinct nucleotide strings.

9. The one or more non-transitory machine-readable information storage medium of claim 8, wherein the prefix comprises one or more trailing zeros and one or more leading zeros in the hash value of the nucleotide string.

**Patentansprüche**

1. Prozessorimplementiertes Verfahren (300), umfassend:

Empfangen (302), über eine Eingabe-/Ausgabeschnittstelle (104), einer Genomsequenz als eine Eingabe, wobei das Genom eine Folge einer Mehrzahl von Nukleotiden ist, durch einen Client;

Teilen, über einen oder mehrere Hardwareprozessoren, der Folge der Mehrzahl von Nukleotiden in eine oder mehrere Skizzen basierend auf einer vordefinierten Skizzengröße, durch den Client;

Schätzen (304), über den einen oder die mehreren Hardwareprozessoren, einer oder mehrerer eindeutiger Nukleotidfolgen für die empfangene Eingabegenomsequenz und einer oder mehrerer Nukleotidfolgen mit Multiplizität unter Verwendung einer datenschutzbewahrenden Kmerlight-Technik, durch den Client;

Hashing (306), über den einen oder die mehreren Hardwareprozessoren, jeder der einen oder mehreren eindeutigen Nukleotidfolgen in jeder der einen oder mehreren Skizzen unter Verwendung einer randomisierten Hash-Funktion, durch den Client, wobei ein Hash-Wert verwendet wird, um einen Abtastpegel und einen Zählerwert einer Nukleotidfolge zu bestimmen;

Durchführen (308), über den einen oder die mehreren Hardwareprozessoren, einer mehrstufigen Abtastung für jede Nukleotidfolge, die das Teilen dieser Folgen in Teilmengen basierend auf der Länge eines Präfixes beinhaltet, durch den Client;

Bestimmen (310), über den einen oder die mehreren Hardwareprozessoren, eines Abtastpegels und eines Zählerwerts jeder Nukleotidfolge basierend auf dem Hash-Wert der Nukleotidfolge, durch den Client;

Verschlüsseln (312), über den einen oder die mehreren Hardwareprozessoren, des Abtastpegels und des Zählerwerts jeder Nukleotidfolge, durch den Client, wobei der verschlüsselte Abtastpegel und der Zählerwert an einen vordefinierten Server gesendet werden;

Vorbereiten einer Matrix des verschlüsselten Abtastpegels und des Zählerwerts für jede der einen oder mehreren Skizzen durch den vordefinierten Server, wobei die Matrix unter Verwendung des verschlüsselten Abtastpegels und Zählerwerts für jede Nukleotidfolge in jeder Skizze unter der einen oder den mehreren Skizzen aktualisiert wird;

Bestimmen (314), über den einen oder die mehreren Hardwareprozessoren, eines optimalen Abtastpegels und eines assoziierten optimalen Zählerwerts für jede Multiplizität in der Matrix, durch den vordefinierten Server;

Bestimmen (316), über den einen oder die mehreren Hardwareprozessoren, einer ungefähren Zählung der einen oder mehreren eindeutigen Nukleotidfolgen für das gegebene Eingabegenom und der einen oder mehreren eindeutigen Nukleotidfolgen mit einer gewissen Multiplizität basierend auf dem bestimmten optimalen Abtastpegel und dem assoziierten optimalen Zählerwert, durch den vordefinierten Server; und

Berechnen (318), über den einen oder die mehreren Hardwareprozessoren, eines Häufigkeitshistogramms auf vollständig homomorphen verschlüsselten genomischen Daten in einer datenschutzbewahrenden Weise basierend auf der bestimmten Approximation der Gesamtanzahl eindeutiger Nukleotidfolgen.

2. Prozessorimplementiertes Verfahren (300) nach Anspruch 1, wobei das Präfix eine oder mehrere nachlaufende Nullen und eine oder mehrere vorlaufende Nullen in dem Hash-Wert der Nukleotidfolge umfasst.

3. Prozessorimplementiertes Verfahren (300) nach Anspruch 1, wobei der vordefinierte Server auf eine Kollisionsbedingung für die verschlüsselten Eingabedaten prüft, um die Matrix des Abtastpegels und Zählerwerts zu aktualisieren.

4. Prozessorimplementiertes Verfahren (300) nach Anspruch 1, wobei der optimale Abtastpegel der Pegel ist, der das höchste Auftreten der Multiplizität in der Matrix aufweist.

5. System (100), umfassend:

eine Mehrzahl von Eingabe-/Ausgabeschnittstellen (104) zum Empfangen einer Genomsequenz als eine Eingabe, wobei das Genom eine Folge einer Mehrzahl von Nukleotiden ist, durch einen Client;

einen Speicher (110) in Kommunikation mit dem einen oder den mehreren Hardwareprozessoren (108), wobei der eine oder die mehreren Hardwareprozessoren konfiguriert sind, um programmierte Anweisungen auszuführen, die in dem Speicher (110) gespeichert sind, um:

die Folge der Mehrzahl von Nukleotiden in eine oder mehrere Skizzen basierend auf einer vordefinierten Skizzengröße zu teilen, durch den Client;

eine oder mehrere eindeutige Nukleotidfolgen für die empfangene Eingabegenomsequenz und eine oder mehrere Nukleotidfolgen mit Multiplizität unter Verwendung einer datenschutzbewahrenden Kmerlight-Technik zu schätzen, durch den Client;

jede der einen oder mehreren eindeutigen Nukleotidfolgen in jeder der einen oder mehreren Skizzen unter Verwendung einer randomisierten Hash-Funktion zu hashen, durch den Client, wobei ein Hash-Wert verwendet wird, um einen Abtastpegel und einen Zählerwert einer Nukleotidfolge zu bestimmen;

eine mehrstufige Abtastung für jede Nukleotidfolge durchzuführen, die das Teilen dieser Folgen in Teilmengen basierend auf der Länge eines Präfixes beinhaltet, durch den Client;

einen Abtastpegel und einen Zählerwert jeder Nukleotidfolge basierend auf dem Hash-Wert der Nukleotidfolge zu bestimmen, durch den Client;

den Abtastpegel und den Zählerwert jeder Nukleotidfolge zu verschlüsseln, durch den Client, wobei der verschlüsselte Abtastpegel und der Zählerwert an einen vordefinierten Server gesendet werden;

eine Matrix des verschlüsselten Abtastpegels und des Zählerwerts für jede der einen oder mehreren Skizzen durch den vordefinierten Server vorzubereiten, wobei die Matrix unter Verwendung des verschlüsselten Abtastpegels und Zählerwerts für jede Nukleotidfolge in jeder Skizze unter der einen oder den mehreren Skizzen aktualisiert wird;

einen optimalen Abtastpegel und einen assoziierten optimalen Zählerwert für jede Multiplizität in der Matrix zu bestimmen, wobei der optimale Abtastpegel der Pegel ist, der das höchste Auftreten einer Multiplizität in der Matrix aufweist, durch den vordefinierten Server;

eine Approximation der einen oder mehreren eindeutigen Nukleotidfolgen für das gegebene Eingabegenom und der einen oder mehreren eindeutigen Nukleotidfolgen mit einer gewissen Multiplizität basierend auf dem bestimmten optimalen Abtastpegel und dem assoziierten optimalen Zählerwert zu bestimmen, durch den vordefinierten Server; und

ein Häufigkeitshistogramm auf vollständig homomorphen verschlüsselten genomischen Daten in einer datenschutzbewahrenden Weise basierend auf der bestimmten Approximation der Gesamtanzahl eindeutiger Nukleotidfolgen zu berechnen.

6. System (100) nach Anspruch 5, wobei das Präfix eine oder mehrere nachlaufende Nullen und eine oder mehrere vorlaufende Nullen in dem Hash-Wert der Nukleotidfolge umfasst.

7. System (100) nach Anspruch 5, wobei der vordefinierte Server auf eine Kollisionsbedingung für die verschlüsselten Eingabedaten prüft, um die Matrix des Abtastpegels und Zählerwerts zu aktualisieren.

8. Ein oder mehrere nichtflüchtige maschinenlesbare Informationsspeichermedien, umfassend eine oder mehrere Anweisungen, die bei Ausführung durch einen oder mehrere Hardwareprozessoren Folgendes bewirken:

Empfangen, über eine Eingabe-/Ausgabeschnittstelle (104), einer Genomsequenz als eine Eingabe, wobei das Genom eine Folge einer Mehrzahl von Nukleotiden ist, durch einen Client;
Teilen, über einen oder mehrere Hardwareprozessoren, der Folge der Mehrzahl von Nukleotiden in eine oder mehrere Skizzen basierend auf einer vordefinierten Skizzengröße, durch den Client;
Schätzen einer oder mehrerer eindeutiger Nukleotidfolgen für die empfangene Eingabegenomsequenz und einer oder mehrerer Nukleotidfolgen mit Multiplizität unter Verwendung einer datenschutzbewahrenden Kmerlight-Technik, durch den Client;
Hashing jeder der einen oder mehreren eindeutigen Nukleotidfolgen in jeder der einen oder mehreren Skizzen unter Verwendung einer randomisierten Hash-Funktion, durch den Client, wobei ein Hash-Wert verwendet wird, um einen Abtastpegel und einen Zählerwert einer Nukleotidfolge zu bestimmen;
Durchführen einer mehrstufigen Abtastung für jede Nukleotidfolge, die das Teilen dieser Folgen in Teilmengen basierend auf der Länge eines Präfixes beinhaltet, durch den Client;
Bestimmen eines Abtastpegels und eines Zählerwerts jeder Nukleotidfolge basierend auf dem Hash-Wert der Nukleotidfolge, durch den Client;
Verschlüsseln des Abtastpegels und des Zählerwerts jeder Nukleotidfolge, durch den Client, wobei der verschlüsselte Abtastpegel und der Zählerwert an einen vordefinierten Server gesendet werden;
Vorbereiten einer Matrix des verschlüsselten Abtastpegels und des Zählerwerts für jede der einen oder mehreren Skizzen durch den vordefinierten Server, wobei die Matrix unter Verwendung des verschlüsselten Abtastpegels und Zählerwerts für jede Nukleotidfolge in jeder Skizze unter der einen oder den mehreren Skizzen aktualisiert wird;
Bestimmen eines optimalen Abtastpegels und eines assoziierten optimalen Zählerwerts für jede Multiplizität in der Matrix, wobei der optimale Abtastpegel der Pegel ist, der das höchste Auftreten einer Multiplizität in der Matrix aufweist, durch den vordefinierten Server;
Bestimmen einer ungefähren Zählung der einen oder mehreren eindeutigen Nukleotidfolgen für das gegebene Eingabegenom und der einen oder mehreren eindeutigen Nukleotidfolgen mit einer gewissen Multiplizität basierend auf dem bestimmten optimalen Abtastpegel und
dem assoziierten optimalen Zählerwert, durch den vordefinierten Server; und
Berechnen eines Häufigkeitshistogramms auf vollständig homomorphen verschlüsselten genomischen Daten in einer datenschutzbewahrenden Weise basierend auf der bestimmten Approximation der Gesamtanzahl eindeutiger Nukleotidfolgen.

9. Ein oder mehrere nichtflüchtige maschinenlesbare Informationsspeichermedien nach Anspruch 8, wobei das Präfix eine oder mehrere nachlaufende Nullen und eine oder mehrere vorlaufende Nullen in dem Hash-Wert der Nukleotidfolge umfasst.

## Revendications

1. Procédé mis en œuvre par processeur (300), comprenant :

la réception (302), via une interface d'entrée/sortie (104), d'une séquence génomique en tant qu'entrée, dans lequel le génome est une chaîne d'une pluralité de nucléotides, par un client ;
la division, via un ou plusieurs processeurs matériels, de la chaîne de la pluralité de nucléotides en un ou plusieurs croquis sur la base d'une taille de croquis prédéfinie, par le client ;
l'estimation (304), via les un ou plusieurs processeurs matériels, d'une ou plusieurs chaînes de nucléotides distinctes pour la séquence génomique d'entrée reçue et d'une ou plusieurs chaînes de nucléotides avec multiplicité en utilisant une technique de kmerlight préservant la confidentialité, par le client ;

le hachage (306), via les un ou plusieurs processeurs matériels, de chacune des une ou plusieurs chaînes de nucléotides distinctes dans chacun des un ou plusieurs croquis en utilisant une fonction de hachage randomisée, par le client, dans lequel une valeur de hachage est utilisée pour déterminer un niveau d'échantillonnage et une valeur de compteur d'une chaîne de nucléotides ;

la réalisation (308), via les un ou plusieurs processeurs matériels, d'un échantillonnage multiniveau pour chaque chaîne de nucléotides qui implique la division de ces chaînes en sous-ensembles sur la base de la longueur d'un préfixe, par le client ;

la détermination (310), via les un ou plusieurs processeurs matériels, d'un niveau d'échantillonnage et d'une valeur de compteur de chaque chaîne de nucléotides sur la base de la valeur de hachage de la chaîne de nucléotides, par le client ;

le cryptage (312), via les un ou plusieurs processeurs matériels, du niveau d'échantillonnage et de la valeur de compteur de chaque chaîne de nucléotides, par le client, dans lequel le niveau d'échantillonnage crypté et la valeur de compteur sont envoyés à un serveur prédéfini ;

la préparation d'une matrice du niveau d'échantillonnage crypté et de la valeur de compteur pour chacun des un ou plusieurs croquis par le serveur prédéfini, dans lequel la matrice est mise à jour en utilisant le niveau d'échantillonnage crypté et la valeur de compteur pour chaque chaîne de nucléotides dans chaque croquis parmi les un ou plusieurs croquis ;

la détermination (314), via les un ou plusieurs processeurs matériels, d'un niveau d'échantillonnage optimal et d'une valeur de compteur optimale associée pour chaque multiplicité dans la matrice, par le serveur prédéfini ;

la détermination (316), via les un ou plusieurs processeurs matériels, d'un compte approximatif des une ou plusieurs chaînes de nucléotides distinctes pour le génome d'entrée donné et des une ou plusieurs chaînes de nucléotides distinctes avec une certaine multiplicité sur la base du niveau d'échantillonnage optimal déterminé et du niveau de compteur optimal associé, par le serveur prédéfini ; et

le calcul (318), via les un ou plusieurs processeurs matériels, d'un histogramme d'abondance sur des données génomiques cryptées entièrement homomorphes d'une manière préservant la confidentialité sur la base de l'approximation déterminée du nombre total de chaînes de nucléotides distinctes.

2. Procédé mis en œuvre par processeur (300) selon la revendication 1, dans lequel le préfixe comprend un ou plusieurs zéros arrière et un ou plusieurs zéros avant dans la valeur de hachage de la chaîne de nucléotides.

3. Procédé mis en œuvre par processeur (300) selon la revendication 1, dans lequel le serveur prédéfini vérifie une condition de collision pour les données d'entrée cryptées pour mettre à jour la matrice du niveau d'échantillonnage et de la valeur de compteur.

4. Procédé mis en œuvre par processeur (300) selon la revendication 1, dans lequel le niveau d'échantillonnage optimal est le niveau qui a l'occurrence la plus élevée de la multiplicité dans la matrice.

5. Système (100) comprenant :

une pluralité d'interfaces d'entrée/sortie (104) pour recevoir une séquence génomique en tant qu'entrée, dans lequel le génome est une chaîne d'une pluralité de nucléotides par un client ;

une mémoire (110) en communication avec les un ou plusieurs processeurs matériels (108), dans lequel les un ou plusieurs processeurs matériels sont configurés pour exécuter des instructions programmées stockées dans la mémoire (110) pour :

diviser la chaîne de la pluralité de nucléotides en un ou plusieurs croquis sur la base d'une taille de croquis prédéfinie, par le client;

estimer une ou plusieurs chaînes de nucléotides distinctes pour la séquence génomique d'entrée reçue et une ou plusieurs chaînes de nucléotides avec multiplicité en utilisant une technique de kmerlight préservant la confidentialité, par le client;

hacher chacune des une ou plusieurs chaînes de nucléotides distinctes dans chacun des un ou plusieurs croquis en utilisant une fonction de hachage randomisée, par le client, dans lequel une valeur de hachage est utilisée pour déterminer un niveau d'échantillonnage et une valeur de compteur d'une chaîne de nucléotides ;

réaliser un échantillonnage multiniveau pour chaque chaîne de nucléotides qui implique la division de ces chaînes en sous-ensembles sur la base de la longueur d'un préfixe, par le client ;

déterminer un niveau d'échantillonnage et une valeur de compteur de chaque chaîne de nucléotides sur la base de la valeur de hachage de la chaîne de nucléotides, par le client ;

crypter le niveau d'échantillonnage et la valeur de compteur de chaque chaîne de nucléotides, par le client, dans lequel le niveau d'échantillonnage crypté et la valeur de compteur sont envoyés à un serveur prédéfini ;

préparer une matrice du niveau d'échantillonnage crypté et de la valeur de compteur pour chacun des un ou plusieurs croquis par le serveur prédéfini, dans lequel la matrice est mise à jour en utilisant le niveau d'échantillonnage crypté et la valeur de compteur pour chaque chaîne de nucléotides dans chaque croquis parmi les un ou plusieurs croquis ;

déterminer un niveau d'échantillonnage optimal et une valeur de compteur optimale associée pour chaque multiplicité dans la matrice, dans lequel le niveau d'échantillonnage optimal est le niveau qui a l'occurrence la plus élevée d'une multiplicité dans la matrice, par le serveur prédéfini ;

déterminer une approximation des une ou plusieurs chaînes de nucléotides distinctes pour le génome d'entrée donné et des une ou plusieurs chaînes de nucléotides distinctes avec une certaine multiplicité sur la base du niveau d'échantillonnage optimal déterminé et du niveau de compteur optimal associé, par le serveur prédéfini ; et

calculer un histogramme d'abondance sur des données génomiques cryptées entièrement homomorphes d'une manière préservant la confidentialité sur la base de l'approximation déterminée du nombre total de chaînes de nucléotides distinctes.

6. Système (100) selon la revendication 5, dans lequel le préfixe comprend un ou plusieurs zéros arrière et un ou plusieurs zéros avant dans la valeur de hachage de la chaîne de nucléotides.

7. Système (100) selon la revendication 5, dans lequel le serveur prédéfini vérifie une condition de collision pour les données d'entrée cryptées pour mettre à jour la matrice du niveau d'échantillonnage et de la valeur de compteur.

8. Un ou plusieurs supports de stockage d'informations lisibles par machine non transitoires comprenant une ou plusieurs instructions qui, lorsqu'elles sont exécutées par un ou plusieurs processeurs matériels, amènent :

la réception, via une interface d'entrée/sortie (104), d'une séquence génomique en tant qu'entrée, dans lequel le génome est une chaîne d'une pluralité de nucléotides, par un client ;

la division, via un ou plusieurs processeurs matériels, de la chaîne de la pluralité de nucléotides en un ou plusieurs croquis sur la base d'une taille de croquis prédéfinie, par le client ;

l'estimation d'une ou plusieurs chaînes de nucléotides distinctes pour la séquence génomique d'entrée reçue et d'une ou plusieurs chaînes de nucléotides avec multiplicité en utilisant une technique de kmerlight préservant la confidentialité, par le client ;

le hachage de chacune des une ou plusieurs chaînes de nucléotides distinctes dans chacun des un ou plusieurs croquis en utilisant une fonction de hachage randomisée, par le client, dans lequel une valeur de hachage est utilisée pour déterminer un niveau d'échantillonnage et une valeur de compteur d'une chaîne de nucléotides ;

la réalisation d'un échantillonnage multiniveau pour chaque chaîne de nucléotides qui implique la division de ces chaînes en sous-ensembles sur la base de la longueur d'un préfixe, par le client ;

la détermination d'un niveau d'échantillonnage et d'une valeur de compteur de chaque chaîne de nucléotides sur la base de la valeur de hachage de la chaîne de nucléotides, par le client ;

le cryptage du niveau d'échantillonnage et de la valeur de compteur de chaque chaîne de nucléotides, par le client, dans lequel le niveau d'échantillonnage crypté et la valeur de compteur sont envoyés à un serveur prédéfini ;

la préparation d'une matrice du niveau d'échantillonnage crypté et de la valeur de compteur pour chacun des un ou plusieurs croquis par le serveur prédéfini, dans lequel la matrice est mise à jour en utilisant le niveau d'échantillonnage crypté et la valeur de compteur pour chaque chaîne de nucléotides dans chaque croquis parmi les un ou plusieurs croquis ;

la détermination d'un niveau d'échantillonnage optimal et d'une valeur de compteur optimale associée pour chaque multiplicité dans la matrice, dans lequel le niveau d'échantillonnage optimal est le niveau qui a l'occurrence la plus élevée d'une multiplicité dans la matrice, par le serveur prédéfini ;

la détermination d'un compte approximatif des une ou plusieurs chaînes de nucléotides distinctes pour le génome d'entrée donné et des une ou plusieurs chaînes de nucléotides distinctes avec une certaine multiplicité sur la base du niveau d'échantillonnage optimal déterminé et du niveau de compteur optimal associé, par le serveur prédéfini ; et

le calcul d'un histogramme d'abondance sur des données génomiques cryptées entièrement homomorphes d'une manière préservant la confidentialité sur la base de l'approximation déterminée du nombre total de chaînes de nucléotides distinctes.

**9.** Un ou plusieurs supports de stockage d'informations lisibles par machine non transitoires selon la revendication 8, dans lesquels le préfixe comprend un ou plusieurs zéros arrière et un ou plusieurs zéros avant dans la valeur de hachage de la chaîne de nucléotides.

100

104-1

104-2

104-N

106

102

108

110

112

**FIG. 1**

**200**

| User-Side Computation | Server-Side Computation |
|---|---|
| User inputs genome sequence | Server uses encrypted row and column numbers from client to update a matrix called sketch |
| For each k-mer in the genome, the hash value is computed to get row and column number | After updating sketch from all k-mers, the minimum row number is obtained for each multiplicity |
| Row number and column number are extracted from hash value and are encrypted and sent to server for further computations | For each multiplicity, the corresponding frequency value is approximated using the given formula in encrypted domain |
| Decrypt each multiplicity to get the corresponding result | |

User Public Key

User Secret Key

FIG. 2

300

Receiving, via an input/output interface, a genome sequence as an input, wherein the genome is a string of a plurality of nucleotides — 302

Dividing the string of the plurality of nucleotides into one or more sketches based on a predefined sketch size — 304

Estimating one or more distinct nucleotide strings for the received input genome sequence and one or more nucleotide strings with multiplicity using a privacy preserving kmerlight technique — 306

Hashing each of the one or more distinct nucleotide strings in each of the one or more sketches using a randomized hash function, wherein the hash value is used to determine a sampling level and a counter value of nucleotide string — 308

Performing a multilevel sampling for each nucleotide string which involves dividing these strings into subsets based on length of a prefix — 310

FIG. 3A

300

| | |
|---|---|
| Determining a sampling level and a counter value of each nucleotide string based on the hash value of the nucleotide | 312 |
| Encrypting the sampling level, and the counter value of each nucleotide string, wherein the encrypted sampling level and the counter value is sent to a predefined server | 314 |
| Determining an optimal sampling level and associated optimal counter value for each multiplicity in the matrix | 316 |
| Determining an approximation of the one or more distinct nucleotide strings for the given input genome and the one or more distinct nucleotide strings with a certain multiplicity based on the determined optimal sampling level and the associated optimal counter level | 318 |
| Computing an abundance histogram on a fully homomorphic encrypted genomic data in privacy preserving manner based on the determined approximation of the total number of distinct nucleotide strings | 320 |

FIG. 3B

**400**

FIG. 4

**FIG. 5A**

**FIG. 5B**

**FIG. 6A**

FIG. 6B

**EP 4 485 258 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- IN 202321043119

### Non-patent literature cited in the description

- Kmerlight: fast and accurate k-mer abundance estimation. **NAVEEN SIVADASAN et al.** ARXIV.ORG. CORNELL UNIVERSITY LIBRARY, 19 September 2016